# EUROPEAN PATENT SPECIFICATION

(11) **EP 1 888 625 B1**
(45) Date of publication and mention of the grant of the patent: **28.10.2009**
(21) Application number: 06744328.3
(22) Date of filing: 26.05.2006
(51) Int. Cl.: C07K 14/445, C07K 19/00, C12N 15/30, C12N 15/62, A61K 39/002, G01N 33/569

(54) **MALARIA VACCINES**
MALARIAIMPFSTOFFE
VACCINS ANTIPALUDIQUES

(30) Priority: 27.05.2005 GB 0510763
(43) Date of publication of application: 20.02.2008
(73) Proprietor: London School of Hygiene and Tropical Medicine, London WC1E 7HT (GB)
(72) Inventor: TETTEH, Kevin K. A., The London School Of Hygiene, London WC1E 7HT (GB); CONWAY, David J., Banjul (GM)
(74) Representative: Irvine, Jonquil Claire
(86) International application number: PCT/GB2006/050126
(87) International publication number: WO 2006/126029

(56) References cited:
- POLLEY SPENCER D ET AL: "Repeat sequences in block 2 of Plasmodium falciparum merozoite surface protein 1 are targets of antibodies associated with protection from malaria." INFECTION AND IMMUNITY, vol. 71, no. 4, April 2003 (2003-04), pages 1833-1842, XP002410607 ISSN: 0019-9567
- CAVANAGH DAVID R ET AL: "Antibodies to the N-terminal block 2 of Plasmodium falciparum merozoite surface protein 1 are associated with protection against clinical malaria" INFECTION AND IMMUNITY, vol. 72, no. 11, November 2004 (2004-11), pages 6492-6502, XP002410608 ISSN: 0019-9567
- TETTEH KEVIN ET AL: "Tackling complex antigenic polymorphism with a minimal polyvalent immunogenic protein." EXPERIMENTAL PARASITOLOGY, vol. 105, no. 1, 2003, page 70, XP002410609 & MAM2004 (MOLECULAR APPROACHES TO MALARIA) CONFERENCE; LORNE, VICTORIA, AUSTRALIA; FEBRUARY 02-05, 2004 ISSN: 0014-4894
- DAVID R. CAVANAGH ET AL.: "A longitudinal study of type-specific antibody responses to Plasmodium falciparum merozoite surface protein-1 in an area of unstable malaria in Sudan" JOURNAL OF IMMUOLOGY, vol. 161, 1998, pages 347-359, XP002410610 & DATABASE UniProt Entry name O43985_PLAFA 1 June 1998 (1998-06-01), CAVANAGH, D:R: ET AL.: "Merozoite surface protein 1 (Fragment)" Database accession no. O43985
- JIANG GANGFENG ET AL: "Sequence diversity of the merozoite surface protein 1 of Plasmodium falciparum in clinical isolates from the Kilombero District, Tanzania" ACTA TROPICA, vol. 74, no. 1, 5 January 2000 (2000-01-05), pages 51-61, XP002410611 ISSN: 0001-706X cited in the application
- CARLOTA DOBAÑO ET AL.: "Identical alleles of Plasmodium falciparum merozoite surface protein 2 found in distant geographic areas and times" PARASITOLOGY INTERNATIONAL, vol. 46, 1997, pages 137-142, XP002410612
- TETTEH KEVIN K A ET AL: "Extensive antigenic polymorphism within the repeat sequence of the Plasmodium falciparum merozoite surface protein 1 block 2 is incorporated in a minimal polyvalent immunogen" INFECTION AND IMMUNITY, vol. 73, no. 9, September 2005 (2005-09), pages 5928-5935, XP002410613 ISSN: 0019-9567

## Description

The present invention relates to malaria vaccines including as an antigenic component a synthetic sequence representing a combination of fragments from the K1 type block 2 repeat region of *Plasmodium falciparum* merozite surface protein 1 (MSP1). The K1 type of the MSP1 block 2 region is highly polymorphic and the K1 type has been found to be the most common of the MSP 1 block 2 region types in African populations. The present invention relates *inter alia* to harnessing the extensive sequence diversity of the K1 type block 2 region of MSP1 for vaccine use in a sequence referred to as the K1 type synthetic repeat (K1SR) sequence. This sequence is capable of inducing antibody responses having wide targeting for MSP1 K1 type block 2 region alleles. To improve breadth of effectiveness as an immunogen against *P. falciparum,* furthermore it can be incorporated into fusion constructs with other *P. falciparum* MSP1 epitopes.

### Background of the invention

Polymorphism in pathogen antigens presents a complex challenge for vaccine design, particularly when diversity is extensive. The N-terminal block 2 region of *Plasmodium falciparum* MSP1 exemplifies high diversity in a pathogen antigen. This region is known to be the target of naturally acquired human antibodies that are associated with a reduced risk of clinical malaria (Conway et al., (2000) Nature Med. 6, 689-692; Polley et al., (2003) Infect. & Immun. 71, 1833-1842; Cavanagh et al., (2004) Infect. & Immun.72, 6492-6502). Hence, there has been interest in priming responses to the MSP 1 block 2 region by vaccination, particularly in young children. However, there are three major allelic types of the MSP1 block 2 region (the K1 type, the MAD20 type and the R033 type) and much subtype polymorphism exists within two of these (K1 and MAD20).

Extensive sequence diversity is exhibited by both the K1 and MAD20 block 2 types within the central tri- and hexa-peptide repeat sequences of each type. The K1 and MAD20 types contain different tri- and hexa-peptide repeat sequences with serine at the first position and variations in the sequence and number of repeats produce subtype allelic differences within each of the types (Conway et al., (2000) Nature Med. 6, 689-692; Miller et al., (1993) Mol. Biochem. Parasitol. 59,1-14). Despite this sequence diversity, most human sera from individuals in malaria endemic regions recognise multiple block 2 allelic variants within a type, and although some individuals have single allele-specific antibody responses to MSP1 block 2, this is less common (Cavanagh et al. (1998) J. Immunol. 161, 347-359; Cavanagh & McBride (1997) Mol. & Biochem. Parasitol. 85,197-211). This can be explained in two ways. Firstly, there are shared type-specific flanking sequences associated with both the K1 and MAD20 block 2 types, which are the target of some antibody responses, and secondly the specific characteristics of the sequence diversity within the K1 and MAD20 types. Four different tripeptides are used in the K1 type and 5 (different) tripeptides are used in the MAD20 type. Thus, all K1 type block 2 alleles contain combinations of the tripeptides SAQ, SGA, SGP and SGT, flanked by common sequences unique to the K1 type of block 2. All MAD20 type block 2 alleles contain combinations of the tripeptides SGG and SVA (with minor variations at the N-terminal end of the repeats using the SVT, SKG and SSG tripeptides), flanked by MAD20 type common sequences. Variation within the K1 and MAD20 block 2 types appears to be restricted to these differences, plus some size constraint on the length of repetitive sequence allowed within the MSP1 molecule (Jiang et al. (2000) Acta Tropica 74, 51-61).

As already noted above, the K1 type is the most common of the types of the block 2 MSP1 region in African populations. For example, the inventors found in a Zambian population that 49 of 91 alleles of *P*. *falciparum* MSP1 block 2 region were of the K1 type and most of these had unique sequences due to variation in tri- and hexa-peptide repetitive motifs. As noted in Polley et al. (2003) ibid, there is therefore particular interest in priming immune responses which broadly target the allelic diversity associated with the repeats of MSP1 K1 type block 2 regions by including repeat motifs from such regions in malaria vaccines.

### Summary of the invention

As an approach to addressing the above-noted need, a minimal length novel polypeptide sequence of 78 amino acid residues has been designed which contains a large proportion of all antibody epitopes within the repeat sequences of the MSP1 K1 type block 2 region (the K1 type synthetic repeat sequence referred to as the K1SR and presented as SEQ. ID. No.1). This was achieved using sera from malaria endemic regions and 23 synthetic peptides representing all the 12 mer amino acid sequences (with the serine of tri-peptides at position 1) derived from a collection of MSP 1 K1 type block 2 repeat sequences present in the GenBank database. The resulting synthetic sequence has been expressed as a recombinant fusion protein having at the N-terminus a glutathione-S-transferase (GST) sequence and in this form has been shown to exhibit broader type specific reactivity with human sera than known individual alleles of the MSP1 K1 type block 2 region.

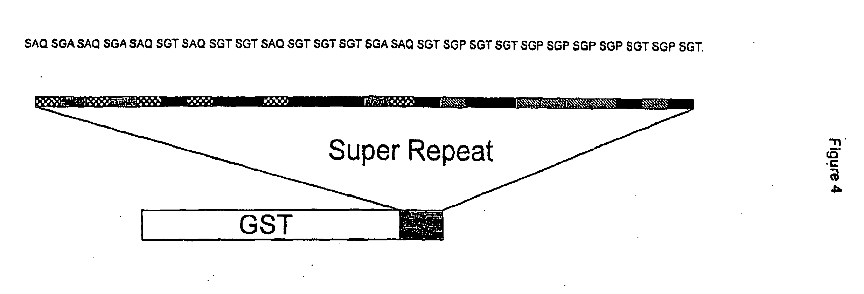

In one aspect, the present invention thus provides a polypeptide comprising the following amino acid sequence as an antigenic sequence (SEQ ID no.1; see also Figure 4):

CTATAGGGCGAATTGGGTACCGGATCCTCTGCACAGTCTGGTGCGTCTGCACAGTCCGGC
---------+---------+---------+---------+---------+---------+
GATATCCCGCTTAACCCATGGCCTAGGAGACGTGTCAGACCACGCAGACGTGTCAGGCCG
S A Q S G A S A Q S G

GCGTCCGCCCAGTCCGGCACCTCTGCCCAGTCCGGCACCTCTGGCACCTCCGCCCAGAGC
---------+---------+---------+---------+---------+---------+
CGCAGGCGGGTCAGGCCGTGGAGACGGGTCAGGCCGTGGAGACCGTGGAGGCGGGTCTCG
A S A Q S G T S A Q S G T S G T S A Q S

GGCACCTCCGGCACCAGTGGCACCTCTGGCGCCTCCGCACAGTCTGGCACCTCCGGCCCG
---------+---------+---------+---------+---------+---------+
CCGTGGAGGCCGTGGTCACCGTGGAGACCGCGGAGGCGTGTCAGACCGTGGAGGCCGGGC
G T S G T S G T S G A S A Q S G T S G P

TCTGGCACCTCCGGCACCTCTGGTCCGTCTGGCCCGTCCGGCCCGAGTGGTCCGAGCGGC
---------+---------+---------+---------+---------+---------+
AGACCGTGGAGGCCGTGGAGACCAGGCAGACCGGGCAGGCCGGGCTCACCAGGCTCGCCG
S G T S G T S G P S G P S G P S G P S G

ACCTCTGGCCCGTCTGGCACCTAAGAATTCCGAGCTCCAGCTTTTGTTCCCTTTAGTGAG
---------+---------+---------+---------+---------+---------+
TGGAGACCGGGCAGACCGTGGATTCTTAAGGCTCGAGGTCGAAAACAAGGGAAATCACTC
T S G P S G T *

G
-
C

The invention also extends to antigenic polypeptides comprising a functional analogue of the K1SR sequence which is of the same length and which retains substantially the same immunogenicity as the K1SR sequence when in the form of a GST-fusion protein, i.e. joined at the N-terminus to a GST sequence. Such a sequence may be any conventional GST sequence as commonly employed for expression and purification of recombinant proteins as fusion proteins, e.g. a GST sequence as encoded by a pGEX expression vector (Smith et al., Single-step purification of polypeptides expressed in Escherichia coli as fusions with glutathione S-transferase (1988) Gene 67, 31-40). It has been found that MSP1 block 2 repeat regions are poorly immunogenic unless conjugated to a carrier protein.
"Substantially the same immunogenicity" may be judged using a panel of different sera or monoclonal antibodies which bind to epitope-presenting fragments spanning the whole K1SR sequence. A variant with "substantially the same immunogencity" will desirably bind with all of the same antibodies or at least about 90 to 99% thereof, preferably at least about 99% thereof. Such a functional analogue may have one or more substitutions, e.g. one or more conservative substitutions, which retain the desired immunogenicity.

A functional analogue as discussed above can be expected to share with the sequence of SEQ. ID. NO.1 ability to bind with monoclonal antibodies to each of the following sequences: (i) SGASAQSG (ii) SAQSGTSGTS and (iii) SAQSGTSGT. Such monoclonal antibodies are exemplified by the monoclonal antibodies Mab 12.2, Mab 123D3 and Mab CE2 respectively as referred to in the exemplification below. Such monoclonal antibodies may be used in initial screening for functional analogues of SEQ. ID. No. 1.

Preferred polypeptides of the invention are fusion proteins wherein a synthetic repeat sequence selected from the K1SR and functional analogues thereof is joined at the N-terminus and /or the C-terminus to an additional amino acid sequence. For example, as indicated above, a K1 type synthetic repeat sequence as described above may be preferably expressed as a fusion protein in which it is joined at the N-terminus to a GST sequence or another amino acid sequence which may be used to aid purification, e.g. an N-terminal sequence to provide a His-tagged protein. Such fusion constructs exhibit broader type-specific reactivity with sera from *P. falciparum* infected individuals than known MSP1 K1 type block 2 alleles, more particularly, for example, the alleles designated 3D7 and Palo Alto.

Of particular interest for vaccine design are fusion constructs in which a K1 type synthetic repeat sequence as described above exhibits immunogenicity, i.e. is capable of raising an immune response to a number of alleles of the MSP1 K1 type block 2 region, and is joined to one or more additional epitope-containing sequences capable of raising an antibody response or a cellular immune response in humans to a *P. falciparum* antigen. Of especial interest are fusion constructs in which a K1 type synthetic repeat sequence of the invention exhibits desirable immunogenicity, e.g. as when joined to an N-terminal GST sequence, and is joined to one or more additional epitope-containing sequences selected from other MSP1-derived sequences, preferably human T cell epitope-containing sequences capable of inducing a cellular immune response to *P*. *fa*/*ciparum* and MSP1 block 2 regions of the types MAD20 and RO33. Preferably, such a hybrid fusion construct may, for example, incorporate, or consist of, for example, all of the following sequences joined consecutively in the N-terminal to C-terminal direction: (i) an N-terminal sequence, for example, to assist with purification such as a GST sequence or a His tag sequence; (ii) a sequence comprising one or more human T cell epitope-presenting sequences derived from an MSP1 K1 type allele such as the 3D7 allele and capable of activating a cellular immune response and (iii) a K1 type synthetic repeat sequence of the invention. Component (ii) may, for example, be a complete K1 type block 1-block 2 allele or a portion or portions thereof. It may comprise an artificial sequence providing a combination of T cell epitopes, e.g. the T cell epitope 1 (T1) and the T cell epitope 2 (T2) derived from the 3D7 block 1- block 2 allele. Such a hybrid sequence may be desirably further extended at the C-terminus by joining to both an MSP1 block 2 region of the MAD20 type and an MSP1 R033 type block 2 region.

In another aspect, the invention provides a composition for inducing an immune response *to P*. *falciparum* including as an antigenic element a K1 type synthetic repeat sequence in the form of a polypeptide or fusion protein as discussed above.

The above-noted aspects of the invention are discussed in more detail below with reference especially to the following sequences and figures.

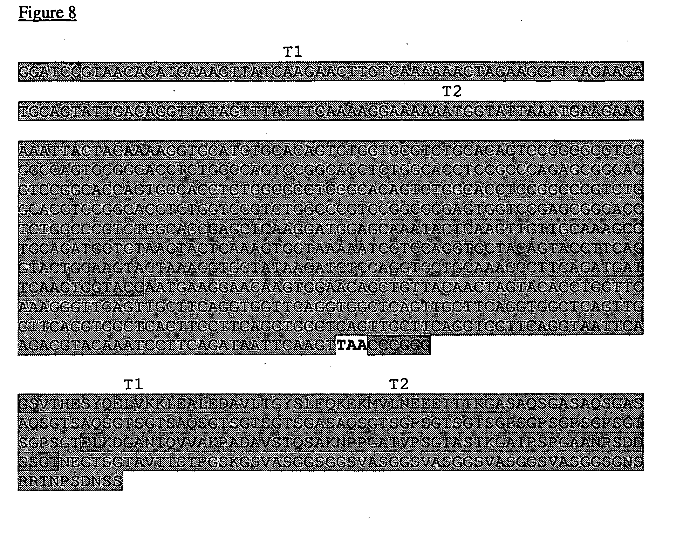

SEQ. ID no. 1: the amino acid sequence (78 amino acid residues) of the invention noted above and designated the K1 type synthetic repeat sequence or K1 SR;
the DNA insert as shown above for cloning of an optimised coding sequence for the K1SR in pPCR-Script Amp and transfer of the coding sequence to the expression vector pGEX-2T for expression of the K1SR as a GST fusion protein in *E*. *coli*; the DNA insert as shown in Figure 8 consisting of the following modules starting at the 5' terminus: (i) BamH1 restriction endonuclease site (GGATTC) encoding GS; (ii) coding sequence for the K1 SR beginning TCT and ending ACC, pre-ceded by a 5' flanking sequence including the T cell epitope 1 (conserved in MSP1) and the T cell epitope 2 (less conserved in MSP1) and follwed by a 3' flanking sequence; (ii) a Sac1 restriction endonuclease site (GAGCTC) encoding EL; (iii) coding sequence for an MSP1 R033 type block 2 allele; (iv) a KpnI restriction site (GGTACC) encoding GT; (v) coding sequence for the Wellcome block 2 allele (an allele of the MAD 20 type); (vi) the stop codon TAA and (vii) a SmaI restriction site (CCCGGG);
the hybrid multi-allelic protein expressed using the DNA insert as shown in Figure 8. The T cell epitopel (T1) sequence and T cell epitope 2 (T2) sequence are underlined.

### Brief description of the figures

The following figures are referred to below in further describing design and construction of the K1 type synthetic repeat sequence noted above and longer fusion constructs containing that synthetic repeat sequence:

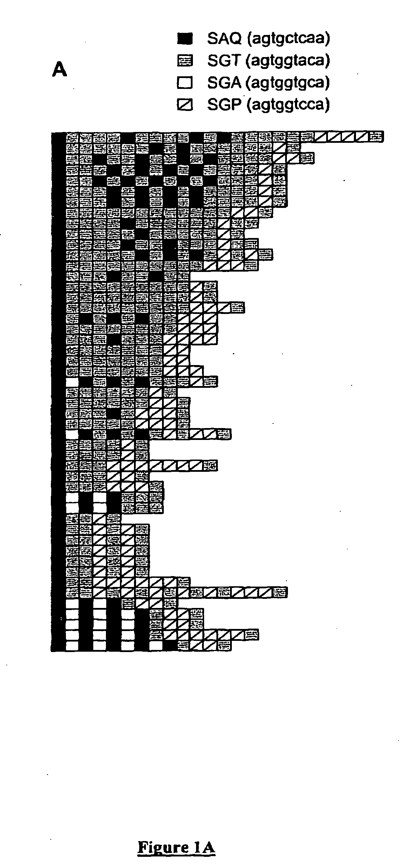

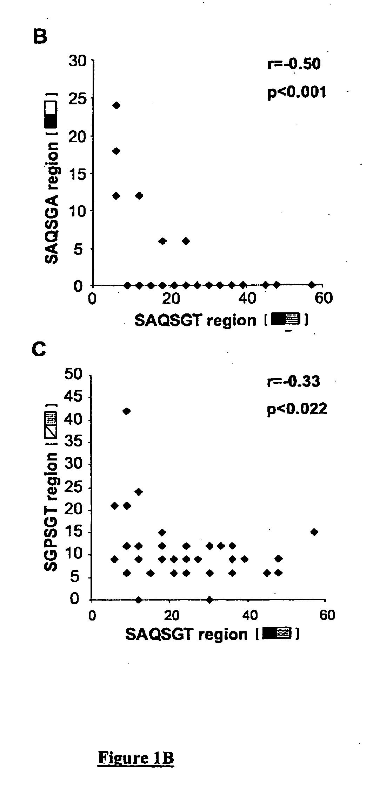

Figure 1. A. Schematic representation of sequences of the repeats of the K1 type of MSP1 block 2 in 49 alleles of this type sampled in Northern Zambia. B. Negative correlation between the amino acid length of sequence in the N-terminal part of the repeats (SAQSGA region) and the central part of the repeats (SAQSGT region). C. Negative correlation between the amino acid length of sequence in the C-terminal part of the repeats (SGPSGT region) and the central part of the repeats (SAQSGT region).

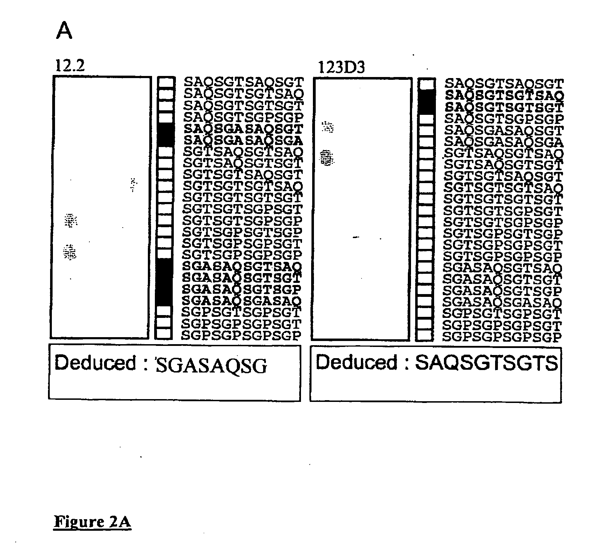

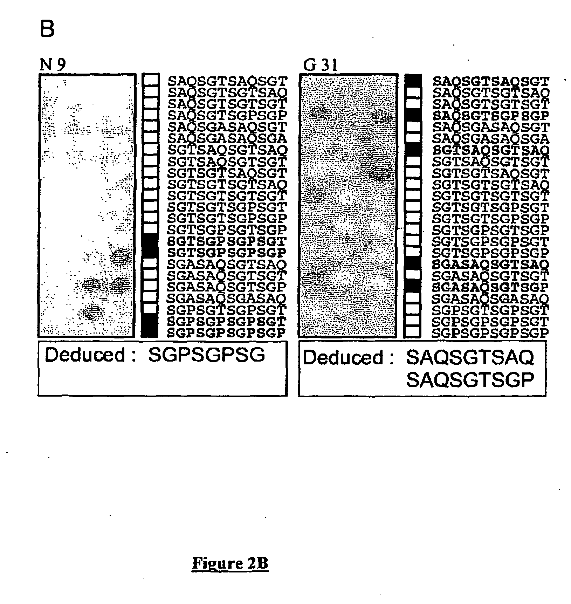

Figure 2. Reactivities of different antibodies against a panel of 23 synthetic peptides representing 12-mer amino acid repeat sequences in the K1 type of MSP1 block 2. Peptides are arrayed in three vertical columns of 8, 8 and 7 spots on the membranes and the identities of these peptide sequences are listed to the right of each membrane (reading downwards for each column of spots and beginning with the left hand column). The sequences corresponding to the spots that are visibly reactive with a given antibody are highlighted in bold print. A. Reactivity of two murine monoclonal antibodies (mabs) known to react with the K1 type of MSP1 block 2. B. Reactivity of two human sera from adults living in endemic areas in Africa.

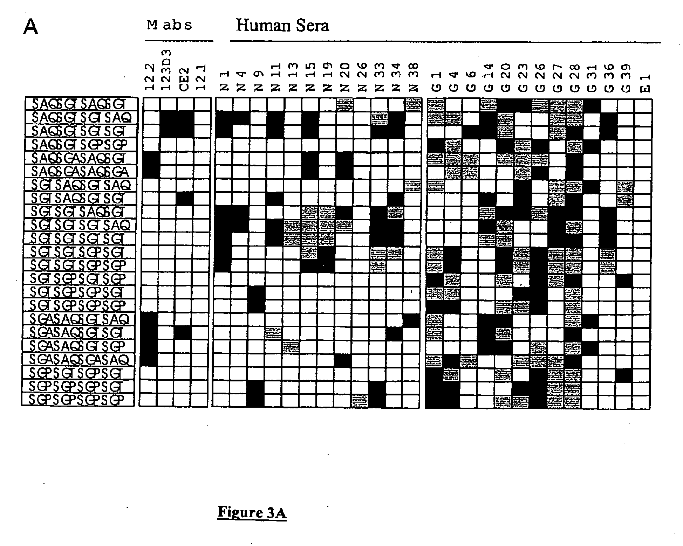

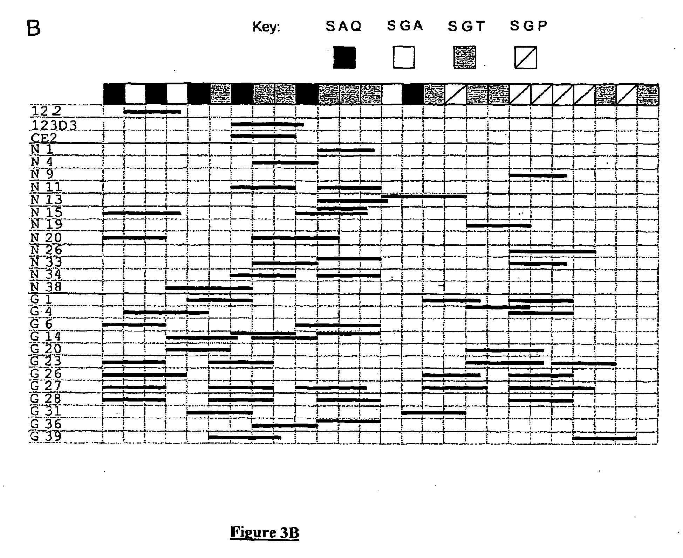

Figure 3. A. Scheme showing the reactivities of murine mabs (12.2, 123D3 and CE2 against MSP1 block 2) and a negative control (12.1 against block 4) and 24 sera from adult Africans (12 from Lagos, Nigeria and 12 from Brefet village in The Gambia) and a representative European control serum (E1) to the chosen 23 synthetic peptides used in arriving at the K1SR B. Deduced specificity of the mabs and the antibodies in each of the human sera given as horizontal lines mapping to different parts of the composite super repeat sequence designated K1 SR as shown schematically.
Figure 4. Figure showing the complete structure of the K1SR as expressed as a GST fusion protein.

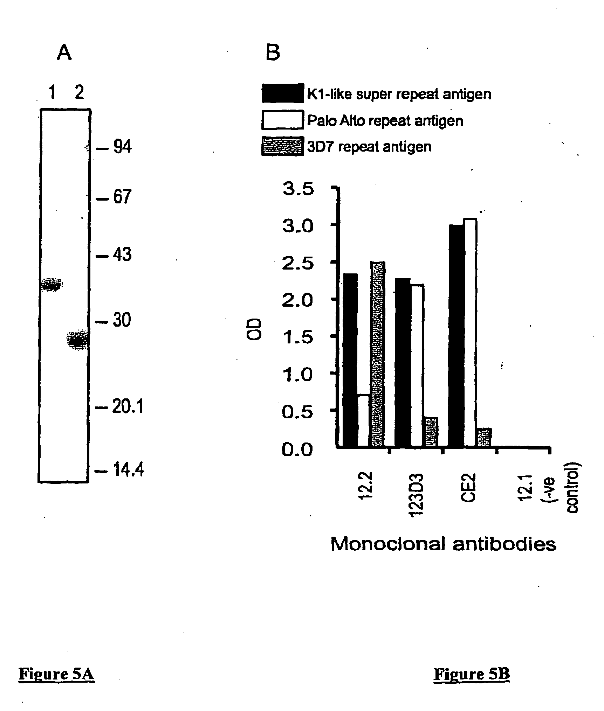

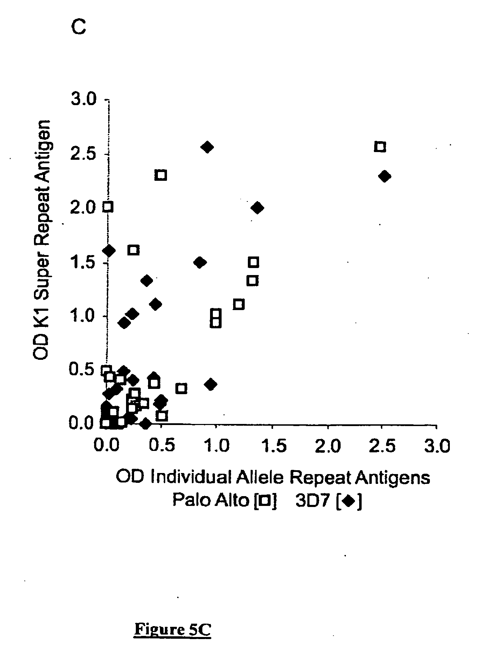

Figure 5. A. Coomassie-stained SDS-PAGE showing expressed GST-K1SR fusion protein (lane 1) and the GST carrier alone (lane 2). B. Mab reactivities to the K1SR by ELISA. C. Scatterplot of ELISA OD values for IgG reactivity to the K1SR and to each of two single allele repeat antigens (Palo Alto and 3D7) in 78 adult West African sera (38 from Nigeria and 40 from The Gambia).

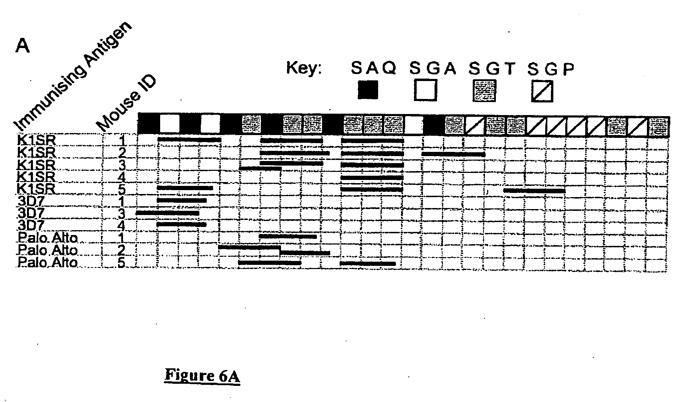

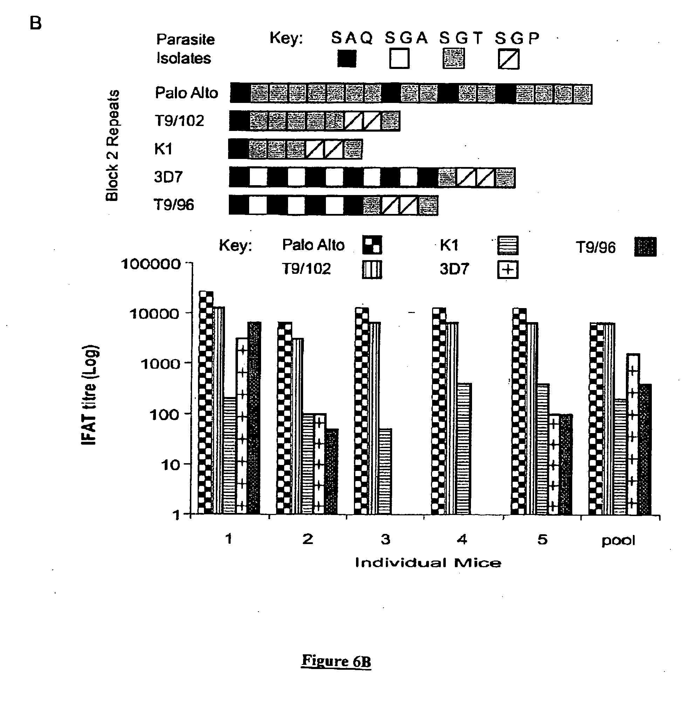

Figure 6. A. Deduced specificity of antibodies from each of five mice immunised with the K1SR and two groups of three mice immunised with single allele repeat antigens (3D7 and Palo Alto) as determined by reactivity with synthetic peptides (the same panel of 23 peptides as described in Figures 2 and 3). B. Deduced amino acid sequences of the MSP1 block 2 repeats in 5 *P*. *falciparum* cultured lines that have K1 type MSP1 block 2 alleles and titres of antibodies in each of the K1SR-immunised mice against schizonts of each of the cultured lines by immunofluorescence.

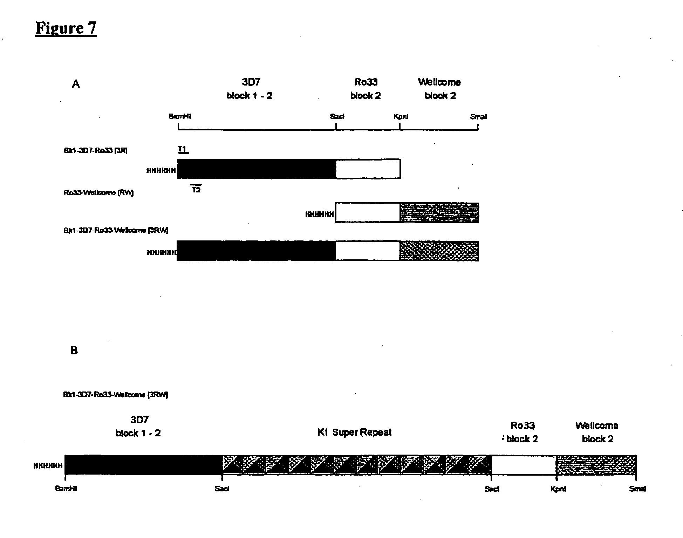

Figure 7. Diagramatic representation of a hybrid construct with an N-terminal His tag incorporating the K1SR preceded by the MSP1 blockl-block 2 3D7 allele (an allele of the K1 type) providing T cell epitopes (Genbank accession no. NP_704838.1; amino acid residues 20 to 133 where amino acid residue 1 is the start methionine residue of the MSP1 sequence) and followed by MSP1 block 2 regions of the R033.and MAD20 types (amino acid residues 54-106 of Genbank accession no. AB116601 and amino acid residues 54 to 118 of Genbank accession no. CAA33163 respectively) (B); (A) diagrammatic representations of corresponding chimeric constructs consisting of joined single MSP 1 alleles and the encoding DNA.
Figures 8: DNA insert in which the different modules as noted above are highlighted in different shades of grey and the corresponding encoded hybrid protein sequence.

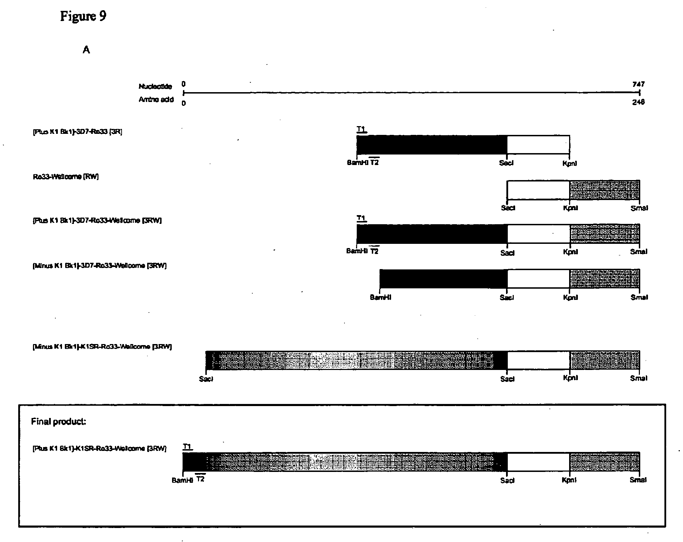

Figure 9: Scheme showing partial constructs corresponding to the hybrid protein shown in Figure 8. Sequences were derived as follows: (a) the T cell epitope 1 and T cell epitope 2 correspond to amino acid residue positions 20 to 29 and 44 to 63 respectively of the MSP1 3D7 block 1- block 2 allele (Genbank accession no. NP_704838.1; amino acid positions 20-133; 3D7 MSP1 block 1, amino acid residues 20-53 and 3D7 MSP1 block 2 (including flanking sequences), amino acid residues 54-133); (b) the K1SR plus 3' flanking sequence; (c) MSP1 RO33 block 2 (Genbank accession no AB116601; amino acid residues 54-106); (d) MSP1 Wellcome block 2 (Genbank accession no. CAA33163; amino acid residues 54-118).
Predicted protein sizes:
Antigen (a): [Plus K1 Bk1/T]-3D7-Ro33 [3R] 16.6 kDa
Antigen (b): R033-Wellcome [RW] 11.0 kDa
Antigen (c): [Plus K1 Bk1/T]-3D7-RO33-Wellcome [3RW] 22.5 kDa
Antigen (d): [Minus K1 Bk1]-3D7-RO33-Wellcome [3RW] 17.3 kDa
Antigen (e): [Minus K1 Bk1]-K1SR-RO33-Wellcome [3RW] 17.8 kDa
Final product: [Plus K1 Bk1/T]-K1SR-RO33-Wellcome [3RW] 22.8 kDa

A construction scheme for the final hybrid protein is given in Example 3.

### Detailed description

### K1 type synthetic repeat sequence

As indicated above, the present invention provides a polypeptide comprising as an antigenic sequence a synthetic sequence designed to contain multiple epitopes that occur amongst different serological subtypes of the highly polymorphic K1 type block 2 region of the *P. falciparum* protein MSP1. More particularly, there is provided such a polypeptide wherein said antigenic sequence is a synthetic repeat sequence selected from the sequence of SEQ. ID. No.1 (the K1SR ; see also Figure 4) and functional analogues thereof of the same length and substantially the same immunogencity as determined in the form of a GST fusion protein. The K1SR barely exceeds the length of naturally-occurring K1 type repeat regions of MSP1. However, in the form of a recombinant GST-fusion protein the sequence has been shown to contain epitopes reacting with all three of Mabs 12.2, 123D3 and CE2 having known specificity for peptides from the K1 type MSP 1 block 2 region and diverse human sera known to react with K1 type MSP1 block 2 repeat sequences. The K1SR of the invention has broader type specific reactivity than individually occurring K1 type MSP1 alleles as illustrated in the exemplification below by reference to comparative studies with the known K1 type MSP1 alleles 3D7 and Palo Alto. In mice, the K1SR as a GST fusion protein has also been shown to induce antibodies that react with a broader set of *P*. *falciparum* parasites than recombinant proteins based on single alleles of the K1 type of MSP1.

The KISR of the invention and functional analogues thereof are thus proposed as highly advantageous sequences for provision in compositions for inducing immune responses to *P. falciparum,* particularly in humans. As indicated above, there is particular interest in such compositions, for example, for priming immune responses to *P. falciparum* in young children in regions where there is high prospective risk of clinical malaria. Such a composition will desirably contain additional epitope-presenting sequences which are capable of inducing immune responses to a *P*. *falciparum* antigen. As indicated above, one or more such sequences may be provided in a fusion construct incorporating the K1SR or a functional analogue thereof. However, such sequences may also be provided as one or more separate polypeptides as further discussed below.

### Fusion proteins containing a K1 type synthetic repeat sequence

As indicated above, a K1 type synthetic repeat sequence of the invention is typically expressed as a recombinant fusion protein. For example, to ease purification a K1 type synthetic repeat sequence may be expressed joined at the N-terminus to a GST sequence or His residue tag sequence. Moreover, as indicated above such a fusion protein may be used directly as an immunogen to induce antibody responses to *P*. *falciparum.*

In a further embodiment, the present invention thus provides a fusion construct in which a K1 type synthetic sequence of the invention is joined at the N-terminus and /or C-terminus to an additional amino acid sequence. The synthetic sequence may, for example, be joined directly or indirectly at the N-terminus to a GST sequence or His tag sequence, e.g. a conventional His6-containing tag sequence. In such a fusion construct, or in fusion construct of the invention with an N-terminal GST or His tag sequence, the K1 type synthetic repeat sequence may be provided as part of a longer hybrid construct containing additional epitope-presenting sequences.

For improvement of breadth of targeting, as indicated above, a K1 type synthetic repeat sequence of the invention may be provided in a fusion protein in the form of a longer antigenic sequence in which it is joined to one or more additional epitope-containing sequences capable of raising an antibody response or a cellular immune response in humans to a *P. falciparum* antigen. Such additional epitope-containing sequences will desirably be *P. falciparum* MSP 1-derived sequences selected from (i) human T cell epitope-containing sequences capable of inducing a cellular immune response to *P. falciparum* (ii) a sequence providing an MSP 1 block 2 repeat region of the MAD 20 type and (iii) a sequence providing an MSP 1 block 2 repeat region of the R033 type. Of especial interest for vaccine design are fusion proteins including all of (i) to (iii) and a K1 type synthetic repeat sequence as described above.

Evidence from previously published work (Parra et al. (2000) Infect. Immun. 68, 2685-2691; Quakyi et al. (1994) J. Immunol. 153, 2082-2092), and from use of computer-based predictive algorithms, has indicated the presence of human T cell epitopes within the flanking sequences of the MSP1 block 2 region, i.e. in the type specific regions preceding and following the block 2 repeats, in the junction region between MSP 1 block 2 and MSP1 block 1 and in MSP1 block 1 itself. Thus, component (i) in a hybrid construct as described above may be a complete MSP 1 K1 type block 1-block 2 allele, such as the MSP1 3D7 block 1- block 2 allele (amino acid residues 20 to 133 of Genbank accession number Nip_704838.1; derived from *P*. *falciparum* 3D7 sequence) or a portion or portions thereof providing one or more human T cell epitopes. As indicated above, it may be an artificial sequence providing a combination of T cell epitope 1 and T cell epitope 2 derived from the same block 1-block 2 allele as illustrated by Figures 8 and 9.

Component (ii) may be one or more MSP 1 MAD 20 type block 2 alleles, e.g. the known Wellcome allele (amino acid residues 54-118 of Genbank accession no. CAA33163).

Component (iii) may desirably be a complete RO33 type MSP 1 block 2 sequence (e.g. amino acid residues 54-106 of GenBank accession no. AB 116601).

Relevant MSP-1 block 2 allele sequences (3D7, Palo Alto, MAD 20, Wellcome and RO33) can also be found in Figure 2 of Cavanagh and McBride (1997) Mol. & Biochem. Parasitol. 85, 197-211: Antigenicity of recombinant proteins derived from *Plasmodium falciparum* merozoite surface protein 1. The same figure also provides the sequence of the MSP I block 1 alleles Palo Alto and MAD 20 which may be used in a hybrid construct as described above.

In a preferred embodiment, a fusion construct is thus provided in which a single K1 type block 1-block 2 allele, e.g. the MSP1 3D7 allele, is joined to the N-terminus of the K1 synthetic repeat sequence. Such a hybrid construct may be desirably further extended, typically at the C-terminus, by addition of an MSP 1 block 2 region of the R033 type and an MSP 1 block 2 region of the MAD 20 type. Such a fusion protein with an N-terminal sequence to aid purification (a His tag sequence) and including both an MSP1 RO33 type block 2 allele and an MSP1 MAD 20 type block 2 allele (the Wellcome allele) is illustrated by Figure 7. The N-terminal sequence may alternatively be a GST sequence. The Wellcome MAD20 type block 2 allele may be substituted by any MAD20 type block 2 allele.

A particularly preferred type of fusion construct of the invention is a fusion construct in which the K1SR is joined at its N- terminus to an artificial sequence providing one or more T cell epitopes derived from an MSP1 K1 type allele, e.g. the T cell epitope 1 and T cell epitope 2 derived from the 3D7 block 1- block 2 allele and is joined at its C-terminus to a sequence providing both an MSP1 block 2 region of the R033 type and an MSP 1 block 2 region of the MAD 20 type, e.g. a complete RO33 type block 2 allele and a complete MAD 20 type allele such as the Welcome allele as illustrated by Figures 8 and 9.

### DNA constructs

In another aspect, the present invention provides polynucleotides, which may be DNAs or RNAs, comprising a coding sequence for a polypeptide or protein of the invention, including fusion constructs as discussed above. As particularly preferred embodiments of DNA according to the invention, there are provided DNAs comprising the K1SR coding sequence as shown in the DNA insert above (nucleotide position 28 to nucleotide position 261). This sequence is codon optimised for expression in *E*. *coli* cells.

The invention also extends to vectors encoding a polypeptide or protein of the invention. The invention additionally provides expression constructs, e.g. expression vectors, in which a DNA coding sequence for a polypeptide or protein of the invention is operably-linked to a promoter sequence and optionally other control sequences. Such expression constructs which are capable of expressing a polypeptide or protein of the invention in human cells may be administered to humans for the purpose of promoting an immune response to *P. falciparum* as further discussed below.

In another aspect of the invention, there is provided a method of producing a polypeptide or protein of the invention which comprises culturing host cells containing an expression vector of the invention under conditions whereby said polypeptide or protein is expressed and isolating said polypeptide or protein. The host cells may be any cells conventionally employed for expression of recombinant proteins including in addition to *E*. *coli* cells, other bacterial cells, yeast cells and mammalian cells. Such *in vitro* host cells containing an expression vector for use in an expression method as described above constitute a still further aspect of the invention.

### Compositions and uses

For administration to induce an immune response to *P. falciparum,* a polypeptide or protein of the invention may be administered to an individual, e.g. a human, together with a pharmaceutically acceptable diluent or adjuvant. Such a composition may take the form of a conventional vaccine composition. It is envisaged that relevant polypeptides or proteins of the invention may, for example, be delivered adsorbed to Alum (Alhydrogel or Adju-Phos, superfos Biosector, Kvistgård, Denmark) or emulsified in the adjuvants Montanide ISA51 or Montanide ISA720 (Seppic, France). Other known adjuvants for human use might be employed. Such adjuvants include AS02 (GSK, Belgium) or MF59 (Chiron, Siena, Italy). However, as indicated above it may be preferred to administer a DNA composition for expression of a polypeptide or protein of the invention *in vivo*. Thus, the invention also provides a composition for inducing a response to *P. falciparum* which comprises an expression construct as described above together with a carrier. Such a composition may, for example, comprise DNA deposited on gold beads in conventional manner for formulation of a DNA vaccine.

A composition of the invention comprising, or capable of expressing in human cells, a K1 type synthetic repeat sequence as described above, may comprise a fusion construct which provides in the cells not only the synthetic repeat sequence but also other epitope-containing sequences as discussed above. However, it will be appreciated that such additional epitope-containing sequences may alternatively be provided by one or more further polypeptides or polynucleotides in the same composition or one or more different compositions.

In a further aspect, the present invention provides use of a polypeptide or protein of the invention or use of a polynucleotide of the invention capable of expressing such a polypeptide or protein in the manufacture of a composition for use in inducing an immune response to *P. falciparum.*

In a still further aspect, there is provided a method of inducing an immune response to *P.falciparum* in an individual, e.g. a human, which comprises administering to said individual a polypeptide or protein of the invention. As will be appreciated from the discussion above, such administration may be direct administration of the polypeptide or protein or indirect administration by expression *in vivo* from a DNA encoding the polypeptide or protein.

### MSP1 block 2 typing

Polypeptides presenting as an antigenic element a K1 type synthetic repeat sequence of the invention and sera raised to such sequences may also be used to type the MSP1 block 2 region in sera from individuals infected with *P. falciparum* and in cultured *P. falciparum* schizonts.

Thus in a further aspect of the invention, there is provided a method of typing the MSP1 block 2 region in a serum from an individual infected with *P. falciparum* which comprises contacting the serum with a polypeptide comprising as an antigenic sequence a K1 type synthetic repeat sequence as discussed above and determining whether antibodies in said serum bind to the presented K1 type synthetic repeat sequence. Such a typing method may conveniently adopt a conventional ELISA format

In a still further aspect the invention provides a method of typing the MSP1 block 2 region in cultured *P. falciparum* schizonts which comprises contacting the schizonts with a serum containing antibodies raised to a K1 synthetic repeat sequence of the invention and determining binding of said antibodies to said schizonts. Antibodies may be raised by immunisation using a fusion protein as discussed above, e.g. by mouse immunisation with the adjuvant Montanide ISA720. Such a typing method may conveniently take the form of an immunofluoresence assay employing secondary labelled antibodies labelled with a fluorescent label such as fluorescein isothiocynate.

The following examples illustrate the invention.

### Examples

### Example 1

### Obtaininig of the K1 type Super Repeat Sequence designated K1SR 1

### Sequencing of P. falciparum MSP1 block 2 region from Zambian samples.

A portion of the *msp1* gene spanning the block 2 region was amplified from genomic DNA isolated from peripheral blood samples of 91 individuals with *P*. *falciparum* infections in Northern Zambia. Polymerase chain reaction primers BK1F and BK3R that annealed to conserved sequences in block 1 and block 3 were used with amplification conditions as described previously (Conway et al., (1998) J. Immunol. 161, 347-359). Amplification products were run and visualised on 2% agarose gels. Allelic sizes of the gene fragment range from approximately 400 - 600 base pairs, and many isolates contain more than one genetic type of *P. falciparum,* so the predominant band was excised for each isolate. This was then purified and DNA sequencing of both strands was performed directly using the BK1F and BK3R primers, using BigDye v3.1 chemistry and electrophoresis on an ABI 377 sequencer (Applied Biosystems). Sequence data for each isolate was visually examined for the quality of every nucleotide using the Sequence Navigator program, with PCR and sequencing reactions being repeated in the case of any uncertainty. Data from the whole population sample were then compiled using the MEGALIGN programe (DNAStar Inc, Madison, WI).

### Results

Out of the 91 alleles of the MSP1 block 2 region which were sequenced, 49 (54%) were of the K1 type, 32 (35%) were of the R033 type and 10 (11%) were of the MAD20 type. Figure 1A shows the repeat sequences of the K1 type alleles revealing 39 different allelic sequences out of 49 sampled. All alleles had the core sequence motif SAQSGT with various repeated permutations of this hexa-peptide or the SGT tri-peptide component. C-terminal to this, all except two of the alleles had the SGPSGT sequence motif with repeated permutations of this hexa peptide or the SGP or SGT tri-peptide components. At the N-terminus of the repeats, some alleles had the SAQSGA motif with repeated permutations of this.

Although the variance in the overall repeat length was considerable (mean amino acid length of repeats = 35.6, variance =146.1), it was much less than the sum of the variance in the length of each of the repeat sub-regions (SAQSGT region, mean = 23.1, variance = 196.6; SGPSGT region, mean = 10.0, variance = 43.6; SAQSGA region, mean = 2.6, variance = 36.0). This was due to significantly non-random negative correlations between the lengths of the different repeat sub-regions in each allele (SAQSGT region vs SAQSGA region, r = -0.50, p < 0.001, Figure 1B; SAQSGT region vs SGPSGT region, r = -0.33, p<0.022, Figure 1C).

It was considered that negative correlations between the lengths of the different sub -regions of the repeats could be due to selection by immune responses specific to each, such that there would be strong selection against alleles with long sequences in more than one sub-region and thus constraint on overall length. To identify epitopes in these repeat sequences, antibodies were assayed against a panel of 23 synthetic peptides (see Table 1 below) representing all of the 12 mer amino acid sequences (with the serine of tri-peptides at position 1) in K1 type repeats of MSP1 block 2 sequences derived globally.

**Table 1**

| **Genbank Accession No.** | **Peptide Sequence** | **Peptide position in MSP 1** |
|---|---|---|
| MSP1PLAFC | SAQSGTSGTSAQ | 64 - 75 |
| | SGTSGTSGTSGT | 67 - 78 |
| AAC69727.1 | SGTSGTSAQSGT | 103 - 114 |
| | SGTSGTSGTSAQ | 100 - 111 |
| AAC69720.1 | SGTSGTSGPSGT | 67 - 78 |
| AAC69733.1 | SGTSGPSGPSGT | 73 - 84 |
| AAC69722.1 | SAQSGTSGTSGT | 64 - 75 |
| AAC69730.1 | SGPSGTSGPSGT | 73 - 84 |
| swall:Q9U4B7 | SGTSGPSGPSGP | 73 - 84 |
| AAC69732.1 | SGTSGPSGTSGP | 75 - 86 |
| swall:25961 | SGTSGTSGPSGP | 87 - 98 |
| AAC69728.1 | SGTSGTSGPSGT | 67 - 78 |
| AAC69721.1 | SGPSGPSGPSGP | 91 - 102 |
| AAC69723.1 | SGPSGPSGPSGT | 97 - 108 |
| AAC69725.1 | SGTSAQSGTSGT | 82 - 93 |
| swall:O4398 | SGTSGTSGPSGT | 67 - 78 |
| swall:O96921 | SAQSGTSGPSGP | 109 - 120 |
| | SGTSAQSGTSAQ | 101 - 111 |
| AAC69718.1 | SAQSGTSAQSGT | 64 - 75 |
| | SGASAQSGTSAQ | 61 - 72 |
| AAC69724.1 | SAQSGASAQSGA | 58 - 69 |
| MSP1PLAFN | SAQSGASAQSGA | 52 - 63 |
| CAA37136.1 | SGASAQSGASAQ | 55 - 66 |
| | SGASAQSGTSGP | 61 - 72 |

### Synthetic peptide assays

### (i) Synthesis of peptides

The above-noted twenty-three peptides were synthesised onto a cellulose solid support (Whatman) using methods as described in Frank, R. (1997) Immunology Methods Manual Vol.2, ed. Lefkovits, I. (Academic Press, pp. 763-795). These peptides were designed to represent all of the deduced 12 mer amino acid sequences contained in the repeat region of all *P. falciparum* K1-like MSP1 block 2 alleles found in the GenBank database and in the above-noted study, starting with a serine at position one (rather than positions two or three) of each tri-peptide repeat (see Table 1). As controls for type-specific reactivity, 24 synthetic peptides of the MAD20-like allelic type (representing all of the known 12 mer repeat sequences starting with a serine) and 12 synthetic peptides of the RO33-like type (all contiguous peptides overlapping by 9 amino acids spanning this non-repeat allelic sequence), were also synthesised and tested with antibodies.

### (ii) Human sera and murine monoclonal antibodies

Sera from seventy eight West African adults, thirty eight (ages 18 to 60) from Lagos in Nigeria and forty adults (ages 22 to 70) from the village of Brefet in The Gambia were employed in ELISA as described below and a subset were employed in synthetic peptide immunoassays. Twenty sera from adults living in the United Kingdom who had never had malaria were used as negative controls. All of these samples were obtained with informed consent under the approval of the relevant local and institutional ethical committees. Four murine monoclonal antibodies were also employed. Specificities of three of these (Mabs 12.2, 123D3 and CE2) for some allelic products of the K1-like type of MSP1 block 2 were known (Locher et al., (1996) Exp. Parasitol. 84, 74-83; Cavanagh & McBride (1997) Mol. Biochem. Parasitol. 85, 197-211) and a control (Mab 12.1) was known to react with MSP1 block 4.

### (iii) ELISAs

50 ng/well of each recombinant antigen (expressed as a GST fusion protein) was coated overnight at 4 °C in 100 µl coating buffer (15 mM Na2CO3, 35 mM NaHCO3, pH 9.3) onto Immulon 4HBX flat bottom microtitre plates (Dynex Technologies Inc). Plates were washed (in PBS with 0.05%Tween 20), blocked (1% skimmed milk in PBS with Tween) for five hours, and washed again. Monoclonal antibodies were diluted as indicated, sera were diluted 1/500, and duplicate 100 µl aliquots in blocking buffer were incubated overnight at 4 °C in antigen-coated wells. The wells were washed, and then incubated with 100 µl of HRP-conjugated rabbit anti-human IgG (at 1/5000) (Dako Ltd.) before detection with O-phenylenediamine /H₂O₂ (Sigma). The mean OD value of each serum-antigen reaction was calculated after correction for binding of the serum to GST alone (this background OD was generally < 0.1). A serum was scored as positive if the corrected OD value was higher than the mean + 3 SD of values for the 20 negative control sera from UK residents. Using selected positive sera, competition ELISAs between pairs of antigens were carried out to define the specificity of the antibodies. The protocol was identical to that for direct ELISA, except that sera diluted 1/500 were pre-incubated with soluble competing antigens at concentrations ranging from 0-10 µg ml⁻¹ for 5 hours before incubation overnight with 50 ng of plate-bound antigen.

### (iv) Peptide Immunoassays

Replicate peptide arrays on cellulose membranes were incubated in blocking solution (TBS/tween-20, 5% sucrose, 2% BSA and 3% skimmed milk powder) at 4 °C overnight. Each membrane was drained on filter paper to remove excess blocking solution and then incubated with a 1/500 dilution of serum (or monoclonal antibodies diluted as specified) in fresh blocking solution and stored at 4 °C. The following day, the membranes were washed twice in each of the following Tris-based solutions, TBS/T-20, TBS/T-20/NaCl, TBS/T-20/Triton X-100 and TBS/T-20. The membranes were blotted dry and then incubated with a 1/5000 dilution ofHRP-conjugated goat anti-human IgG (DAKO) in blocking solution for 4 hours. The membranes were washed twice in each of the above solutions followed by two washes in TBS. The membranes were blotted dry of excess buffer and results visualised after development in stabilised TMB substrate (Promega) for 2 mins.

### Results

Each of the three Mabs 12.1, 123D3 and CE2 had a different profile of reactivity with the K1-like synthetic peptides (Figure2A: Mab 12.2 reacted with six peptides, Mab 123D3 reacted with two, and Mab CE2 reacted with four). None reacted with any of the MAD20-like or RO33-like peptide controls and the negative control antibody (Mab 12.1 against block 4 of MSP1) was not reactive with any of the peptides. From examining the reactivity profiles, the minimal primary sequence specificities were deduced as SGASAQSG for Mab 12.2, SAQSGTSGTS for Mab 123D3, and SAQSGTSGT for Mab CE2. The peptides were designed to have serine at the first position of each tri-peptide repeat rather than the second or third, so it is not known whether the terminal one or two amino acids are essential in the case of some deduced specificities.

Twelve sera from Nigerian adults and twelve from Gambian adults that had antibodies to MSP1 block 2 recombinant proteins as assayed by ELISA were then tested to see whether the specificities of human antibodies could be deduced with the synthetic peptide array. Each serum reacted with between three and nine different peptides. Reactivities of two of the sera are shown in Figure 2B and scored results of all are given in Figure 3A. From each of these reactivity profiles, between one and three distinct specificities were deduced to be present in each serum (for a few sera there was also evidence of additional weaker reactivities). A composite sequence (SEQ. ID. no. 1) was designed that encompassed the different deduced specific specificities in the minimal overall sequence length (see Figure 3B and Figure 4). All the deduced epitopes were mapped onto a sequence of 78 amino acids (containing 26 tri-peptides) which was only slightly longer than the longest naturally-occuring individual K1 type MSP1 block 2 repeat that has been described (Ferreira et al., (2003) Gene 304, 65-75).

### Expression of the composite sequence

To express the composite of repeats as an antigen, a novel DNA sequence was constructed and cloned into a pGEX plasmid for expression as a GST fusion protein in *E. coli.*

First, a synthetic gene sequence (SEQ. ID no. 2) encoding the designed super repeat construct was assembled from synthetic oligonucleotides for cloning into pPCR-Script Amp vector (GeneArt, Regensburg, Germany) KpnI and SacI restriction sites. This sequence, which provided a codon-optimised coding sequence for expression in *E.coli* and terminal Bam H1 and EcoRI sites, was subcloned from the pPCR-Script Amp vector into pGEX-2T (Amersham Pharmacia Biotech) for expression of the K1 type synthetic repeat sequence as a GST-fusion protein in BL21 (DE3) *E.coli* cells. Expression and purification followed the manufacturer's protocols as described previously for other MSP1 block 2 recombinant proteins (Polley et al., (2003) Infect. Immun. 71, 1833-1842; Cavanagh & McBride (1997) Mol. Biochem. Parasitol. 85, 197-211). The K1SR was expressed abundantly in soluble form and purified on a glutathione column (Figure 5A). The recombinant antigen reacted specifically with each of the 3 Mabs against epitopes in the K1-like repeat sequences (Figure 5B) and with Nigerian and Gambian adult sera (Figure 5C). Significantly, the K1SR was found to show a broader reactivity with these antibodies than antigens representing individual alleles (Figures 5B and 5C).

### Mouse immunisations

Five MF1 outbred mice were immunised with the K1SR recombinant protein following a protocol used previously for immunisation with other MSP1 block 2 recombinant proteins (Polley et al., (2003) Infect. Immun. 71, 1833-1842; Cavanagh & McBride (1997) Mol. Biochem. Parasitol. 85, 197-211). All animals were given three 50 µg doses of purified protein in the adjuvant ImjectAlum (Pierce) at monthly intervals; serum was collected before immunisation and 12-14 days after each dose.

### Results

Mice immunised with the K1SR recombinant protein produced antibodies to different primary sequence determinants as determined by peptide immunoassay (Figure 6A). Interestingly, all five mice produced antibodies against the SGTSGTSGT epitope, although the remaining antibody profile differed between each animal. As a comparison, sera from mice previously immunised with distinct K1 type repeat antigens (3D7 and Palo Alto) were also tested against the panel of K1-like synthetic peptides and demonstrated a narrower range of specific reactivities (Figure 6A). Mice immunised with the 3D7 repeat sequence antigen showed a profile of reactivity encompassing the SAQSGASAQ and SGASAQS motifs (in the N-terminal part of the repeat array). The mice immunised with the Palo Alto repeat sequence antigen reacted with the more central SAQSGTSAQ, SGTSGT and SGTSAQSGT motifs (Figure 6A). In contrast, the mice immunised with the K1 type Super Repeat Sequence recognised more diverse repeat sequence epitopes located in the N-terminal, central, and C-terminal subregions.

### Immunofluorescence Assays (IFAs)

Antibody reactivity was then tested against a panel of 5 *P. falciparum* cultured lines with different repeat sequences of the K1-like MSP1 block 2 (Figure 6B), by immunofluorescence with parasite schizonts. The IFA was performed as described for sera raised to other MSP1 block 2 antigens (Polley et al., (2003) Infect. Immun. 71, 1833-1842; Cavanagh & Mc bride (1997) Mol. Biochem. Parasitol. 85, 197-211). Acetone-fixed multiwell slides of schizonts from five *P. falciparum* cultured lines (Palo Alto, 3D7, T9/96, T9/102 and K1) were probed with the murine sera raised against the K1SR (at doubling dilutions from 1/50 up to 1/51200). Endpoint titres were stringently determined and expressed as the highest dilution that still gave clear schizont-specific fluoresence (scored as '++', on a '+' to '++++' visual scoring scheme).

### Results

All mice produced antibodies with endpoint titres of at least 1/6400 against schizonts of at least one of the cultured lines. The profile of reactivity to different parasites varied among the mice (Figure 6B) in a manner consistent with the deduced specificity of the antibodies to particular repeat sequences (Figure 6A). For example, most mice sera recognised all the parasite lines, although the sera of mice 3 and 4 did not recognise T9/96 and 3D7 (Figure 6B), parasite lines that did not have the primary sequence motifs that were defined for the antibodies of those mice (Figure 6A).

### Example 2: Construction of tandem array fusion proteins containing the K1SR 1

DNA was obtained from *P. falciparum* clone 3D7 and *P. falciparum* isolates RO33 and Wellcome maintained by the WHO Registry of Standard Strains of Malaria Parasites at the University of Edinburgh. A recombinant DNA with BamH1 and Sma I ends was inserted into the vector pQE30 so as to provide a sequence encoding an N-terminally His tagged tandem array fusion protein consisting of the following components from the N to C-terminus:
(a) a His tag of 6 His residues encoded by the vector;
(b) a MSP1 3D7 block 1- block 2 allele (Genbank accession no. NP_704838.1; amino acid positions 20-133; 3D7 MSP1 block 1, amino acid residues 20-53 and 3D7 MSP1 block 2 allele (including flanking sequences), amino acid residues 54 - 133.)
(c) the K1 type synthetic repeat sequence;
(d) the MSP1 RO33 block 2 allele (accession number AB116601; amino acid residues 54-106; whole allele including flanking sequences) and
(e) the MSP1 Wellcome block 2 allele (accession number CAA33163; amino acid positions 54-118; MAD 20 type; whole allele including flanking sequences.)

Element (b) provided recognised human T cell epitopes (T cell epitope 1 (T1) at amino acid positions 20-29; T cell epitope [T2] at amino acid positions 44 to 63).

Corresponding partial constructs comprising combinations of single alleles and corresponding encoding DNA inserts are shown diagrammatically in Figure 7.

### Example 3: construction of the multi-allelic hybrid construct shown in Figure 8

### Part 1

### Generation of the K1SR fused to both the 5' and 3' K1 type flanking sequences

### Step 1: Generation of N-terminal flanking region and T cell epitopes

Two long oligonucleotides [5'flank hyb R1 + 5' flank hyb F1] covering both desired T cell epitopes and the block 2 flanking region were fused together yielding a 163 bp product using a PCR protocol.

### Step 2: Fusing the K1SR to the 3' flanking region

A K1SR forward primer was used in conjunction with a 3' primer (3' flank R1) which was designed with a K1SR complementary overhang to fuse seamlessly using PCR.

### Step 3: Fusing the 5'flanking/T cell epitopes to the K1SR + 3'flanking region

The purified products from Steps 1 and 2 can be combined in a PCR reaction and fused together using the primers 5' flank hyb R1 and 3' flank R1.

### Part 2

### Step 4: verification of products

Each of the products generated in Steps 1-3 can be cloned into the pGEMT-easy vector and sequence verified, using both vector specific primers and insert specific primers ( where appropriate).

### Step 5: construction of the final product ( T-K1SR-RO33-Wellcome)

Once each module has been sequence verified, the K1 SR + flanking insert can be restriction digested and ligated into a his tag expression vector with the R033 and Wellcome allele modules already in place.

The immunological characteristics of the final construct may be compared with those of the partial constructs shown in Figure 9

## Claims

1. A polypeptide comprising an antigenic sequence derived from repeat regions of *Plasmodium falciparum* K1 type MSP1 block 2 alleles, wherein said antigenic sequence is selected from the sequence of SEQ. ID no.1 (designated the K1 type synthetic repeat sequence 1; KISR): 
Ser Ala Gln Ser Gly Ala Ser Ala Gln Ser Gly Ala Ser Ala Gln Ser
1               5                   10                  15

Gly Thr Ser Ala Gln Ser Gly Thr Ser Gly Thr Ser Ala Gln Ser Gly
            20                  25                  30

Thr Ser Gly Thr Ser Gly Thr Ser Gly Ala Ser Ala Gln Ser Gly Thr
        35                  40                  45

Ser Gly Pro Ser Gly Thr Ser Gly Thr Ser Gly Pro Ser Gly Pro Ser
    50                  55                  60

Gly Pro Ser Gly Pro Ser Gly Thr Ser Gly Pro Ser Gly Thr
65                  70                  75
 and functional analogues thereof of the same length and substantially the same immunogenicity as determined in the form of a fusion protein joined at the N-terminus to an N-terminal glutathione-S-transferase (GST) sequence.

2. A fusion protein comprising a polypeptide as claimed in claim 1 in which said antigenic sequence is joined at the N-terminus and/or the C-terminus to an additional amino acid sequence.

3. A fusion protein as claimed in claim 2 in which said antigenic sequence is joined directly or indirectly at the N-terminus to a GST sequence or His tag sequence.

4. A fusion protein as claimed in claim 2 or claim 3 wherein said antigenic sequence is joined to one or more additional epitope-containing sequences capable of raising an antibody response or cellular immune response in humans to a *P. falciparum* antigen.

5. A fusion protein as claimed in claim 4 wherein said additional epitope-containing sequences are *P. falciparum* MSP 1-derived sequences selected from (i) human T cell epitope-containing sequences capable of inducing a cellular immune response to *P. falciparum*; (ii) a sequence providing an MSP1 block 2 repeat region of the MAD 20 type and (iii) a sequence providing an MSP 1 block 2 repeat region of the RO33 type.

6. A fusion protein as claimed in claim 5 comprising a hybrid construct wherein an antigenic sequence as defined in claim 1 is joined at the N-terminus to a sequence providing one or more human T cell epitopes derived from an MSP1 allele.

7. A fusion protein as claimed in claim 6 comprising a hybrid construct consisting of (i) an MSP1 K1 type block 1-block 2 allele and (ii) an antigenic sequence as defined in claim 1.

8. A fusion protein as claimed in claim 6 or claim 7 wherein said hybrid construct is further extended by addition of a sequence providing an MSP1 block 2 region of the RO33 type and an MSP 1 block 2 region of the MAD 20 type.

9. A fusion protein as claimed in claim 8 wherein said hybrid construct consists of the following components in the N- to C-terminal direction: (i) an MSP1 K1 type block 1-block 2 allele; (ii) an antigenic sequence as defined in claim 1; and (iii) a sequence providing an MSP1 block 2 region of the RO33 type and a MSP1 block 2 region of the MAD 20 type.

10. A fusion protein as claimed in claim 8 wherein said hybrid construct consists of the following components in the N- to C-terminal direction: (i) a sequence providing one or more human T cell epitopes derived from an MSP1 K1 type allele; (ii) an antigenic sequence as defined in claim 1 and (iii) a sequence providing an MSP1 block 2 region of the RO33 type and an MSP1 block 2 region of the MAD 20 type.

11. A fusion protein as claimed in any one of claims 6 to 10 in which said hybrid construct is further joined at the N-terminus to an N-terminal GST sequence or His tag sequence.

12. A polynucleotide which encodes a polypeptide or protein according to any one of the preceding claims.

13. A polynucleotide as claimed in claim 12 which is a DNA.

14. A vector comprising a polynucleotide as claimed in claim 13.

15. A polynucleotide as claimed in claim 13 in which the coding sequence for said polypeptide or protein is operably-linked to a promoter sequence.

16. An expression vector comprising a polynucleotide as claimed in claim 15.

17. A method of producing a polypeptide or protein as claimed in any one of claims 1 to 11 which comprises culturing host cells containing an expression vector according to claim 16 under conditions whereby said polypeptide or protein is expressed and isolating said polypeptide or protein.

18. An *in vitro* host cell containing an expression vector according to claim 16.

19. A composition for inducing an immune response to *P. falciparum* which comprises a polypeptide or protein according to any of claims 1 to 11 together with a pharmaceutically acceptable diluent or adjuvant.

20. A composition for inducing an immune response to *P. falciparum* which comprises a polynucleotide according to claim 15 or an expression vector according to claim 16 together with a carrier.

21. Use of a polypeptide or protein as claimed in any one of claims 1 to 11, a polynucleotide as claimed in claim 15, or an expression vector as claimed in claim 16, in the manufacture of a composition for use in inducing an immune response to *P*. *falciparum.*

22. A method of typing the MSP 1 block 2 region in a serum from an individual infected with *P. falciparum* which comprises contacting said serum with a polypeptide comprising an antigenic sequence as defined in claim 1 and determining whether antibodies in said serum bind to the said sequence.

23. A method of typing the MSP 1 block 2 region in cultured *P. falciparum* schizonts which comprises contacting the schizonts with a serum containing antibodies raised to an antigenic sequence as defined in claim 1 and determining binding of said antibodies to said schizonts.

## Patentansprüche

1. Polypeptid, das eine von Wiederholungsbereichen von *Plasmodium falciparum* K1 Typ MOP1 Block 2 -Allelen abgeleitete Antigensequenz aufweist, wobei die Antigensequenz aus der Sequenz mit der SEQ. ID-Nr 1 ausgewählt ist (bezeichnet als synthetische Wiederholungssequenz 1 vom Typ K1; KISR): 
Ser Ala Gln Ser Gly Ala Ser Ala Gln Ser Gly Ala Ser Ala Gln Ser
1               5                   10                  15
Gly Thr Ser Ala Gln Ser Gly Thr Ser Gly Thr Ser Ala Gln Ser Gly
            20                  25                  30
Thr Ser Gly Thr Ser Gly Thr Ser Gly Ala Ser Ala Gln Ser Gly Thr
        35                  40                  45
Ser Gly Pro Ser Gly Thr Ser Gly Thr Ser Gly Pro Ser Gly Pro Ser
    50                  55                  60
Gly Pro Ser Gly Pro Ser Gly Thr Ser Gly Pro Ser Gly Thr
65                  70                  75
 und funktionelle Analoge davon mit der gleichen Länge und im wesentlichen der gleichen Immunogenität, bestimmt in Form eines Fusionsproteins, das am N-Terminus an eine N-terminale Glutathion-S-Transferase-(GST-)Sequenz gekoppelt ist.

2. Fusionsprotein, das ein Polypeptid nach Anspruch 1 aufweist, in dem die Antigensequenz am N-Terminus und/oder am C-Terminus an eine weitere Aminosäuresequenz gekoppelt ist.

3. Fusionsprotein nach Anspruch 2, in dem die Antigensequenz am N-Terminus direkt oder indirekt an eine GST-Sequenz oder eine His-tag-Sequenz (Sequenz mit His-Anhang) gekoppelt ist.

4. Fusionsprotein nach Anspruch 2 oder Anspruch 3, wobei die Antigensequenz an eine oder mehrere epitophaltige Sequenzen gekoppelt ist, die eine Antikörperreaktion oder zelluläre Immunreaktion auf ein *P*. *falciparum*-Antigen bei Menschen hervorrufen können.

5. Fusionsprotein nach Anspruch 4, wobei die zusätzlichen epitophaltigen Sequenzen von *P*. *falciparum* MSP1 abgeleitete Sequenzen sind, die unter (i) humanes T-Zellen-Epitop enthaltenden Sequenzen, die eine zelluläre Immunreaktion auf *P. falciparum* auslösen können; (ii) einer Sequenz, die einen MSP1 Block 2-Wiederholungsbereich vom Typ MAD 20 bereitstellt; und (iii) einer Sequenz, die einen MSP1 Block 2-Wiederholungsbereich vom Typ RO33 bereitstellt, ausgewählt sind.

6. Fusionsprotein nach Anspruch 5, das ein Hybridkonstrukt aufweist, wobei eine Antigensequenz gemäß der Definition in Anspruch 1 am N-Terminus an eine Sequenz gekoppelt ist, die ein oder mehrere, von einem MSP1-Allel abgeleitete humane T-Zellen-Epitope bereitstellt.

7. Fusionsprotein nach Anspruch 6, das ein Hybridkonstrukt aufweist, das aus (i) einem MSP1-Allel vom Typ K1 Block 1 und (ii) einer Antigensequenz gemäß der Definition in Anspruch 1 besteht.

8. Fusionsprotein nach Anspruch 6 oder Anspruch 7, wobei das Hybridkonstrukt durch Addition einer Sequenz weiter verlängert wird, die einen MSP1 Block 2-Bereich vom Typ R033 und einen MSP1 Block 2-Bereich vom Typ MAD 20 bereitstellt.

9. Fusionsprotein nach Anspruch 8, wobei das Hybridkonstrukt in der Richtung vom N- zum C-Terminus aus den folgenden Komponenten besteht: (i) einem MSP1 K1-Allel vom Typ K1 Block 1-Block 2; (ii) einer Antigensequenz gemäß der Definition in Anspruch 1; und (iii) einer Sequenz, die einen MSP1 Block 2-Bereich vom Typ RO33 und einen MSP1 Block 2-Bereich vom Typ MAD 20 bereitstellt.

10. Fusionsprotein nach Anspruch 8, wobei das Hybridkonstrukt in Richtung vom N- zum C-Terminus aus den folgenden Komponenten besteht: (i) einer Sequenz, die ein oder mehrere humane T-Zellen-Epitope bereitstellt, die von einem MSP1-Allel vom Typ K1 abgeleitet sind; (ii) einer Antigensequenz gemäß der Definition in Anspruch 1; und (iii) einer Sequenz, die einen MSP1 Block 2-Bereich vom Typ RO33 und einen MSP1 Block 2-Bereich vom Typ MAD 20 bereitstellt.

11. Fusionsprotein nach einem der Ansprüche 6 bis 10, wobei das Hybridkonstrukt ferner am N-Terminus an eine N-terminale GST-Sequenz oder His-tag-Sequenz gekoppelt ist.

12. Polynucleotid, das ein Polypeptid oder Protein nach einem der vorstehenden Ansprüche codiert.

13. Polynucleotid nach Anspruch 12, das eine DNA ist.

14. Vektor, der ein Polynucleotid nach Anspruch 13 aufweist.

15. Polynucleotid nach Anspruch 13, in dem die Codierungssequenz für das Polypeptid oder Protein funktionsfähig an eine Promotorsequenz gekoppelt ist.

16. Expressionsvektor, der ein Polynucleotid nach Anspruch 15 aufweist.

17. Verfahren zur Herstellung eines Polypeptids oder Proteins nach einem der Ansprüche 1 bis 11, das aufweist: Kultivieren von Wirtszellen, die einen Expressionsvektor nach Anspruch 16 enthalten, unter Bedingungen, durch die das Polypeptid oder Protein exprimiert wird, und Isolieren des Polypeptids oder Proteins.

18. In-vitro-Wirtszelle, die einen Expressionsvektor nach Anspruch 16 enthält.

19. Zusammensetzung zur Auslösung einer Immunantwort auf P. falciparum, die ein Polypeptid oder Protein nach einem der Ansprüche 1 bis 11 zusammen mit einem pharmazeutisch akzeptierbaren Verdünnungsmittel oder Adjuvans aufweist.

20. Zusammensetzung zur Auslösung einer Immunantwort auf P. falciparum, die ein Polynucleotid nach Anspruch 15 oder einen Expressionsvektor nach Anspruch 16 zusammen mit einem Träger enthält.

21. Verwendung eines Polypeptids oder Proteins nach einem der Ansprüche 1 bis 11, eines Polynucleotids nach Anspruch 15 oder eines Expressionsvektors nach Anspruch 16 bei der Herstellung einer Zusammensetzung zur Verwendung bei der Auslösung einer Immunantwort auf P. falciparum.

22. Verfahren zur Typisierung des MSP 1 Block 2-Bereichs in einem Serum von einer mit *P*. *falciparum* infizierten Person, wobei das Verfahren aufweist: Inkontaktbringen des Serums mit einem Polypeptid, das eine Antigensequenz gemäß der Definition in Anspruch 1 aufweist, und Ermitteln, ob Antikörper in dem Serum an die Sequenz binden.

23. Verfahren zur Typisierung des MSP 1 Block 2- Bereichs in kultivierten P. falciparum-Schizonten, wobei das Verfahren aufweist: Inkontaktbringen der Schizonten mit einem Serum, das zu einer Antigensequenz gemäß der Definition in Anspruch gezogene Antikörper enthält, und Ermitteln der Bindung der Antikörper an die Schizonten.

## Revendications

1. Polypeptide comprenant une séquence antigénique dérivée des régions de répétition des allèles MSP1 bloc 2 du type *Plasmodium falciparum* K1, dans lequel ladite séquence antigénique est choisie parmi la séquence de SEQ. ID n° 1 (désignée la séquence de répétition synthétique 1 du type K1; KISR): 
Ser Ala Gln Ser Gly Ala Ser Ala Gln Ser Gly Ala Ser Ala Gln Ser
1               5                   10                  15
Gly Thr Ser Ala Gln Ser Gly Thr Ser Gly Thr Ser Ala Gln Ser Gly
            20                  25                  30
Thr Ser Gly Thr Ser Gly Thr Ser Gly Ala Ser Ala Gln Ser Gly Thr
        35                  40                  45
Ser Gly Pro Ser Gly Thr Ser Gly Thr Ser Gly Pro Ser Gly Pro Ser
    50                  55                  60
Gly Pro Ser Gly Pro Ser Gly Thr Ser Gly Pro Ser Gly Thr
65                  70                  75
 et des analogues fonctionnels de celui-ci de la même longueur et substantiellement de la même immunogénicité tel que déterminé sous la forme d'une protéine de fusion jointe au niveau de la terminaison N à une séquence glutathione-S-transférase (GST) N-terminale.

2. Protéine de fusion comprenant un polypeptide tel que revendiqué selon la revendication 1, dans laquelle ladite séquence antigénique est jointe au niveau de la terminaison N et/ou de la terminaison C à une séquence d'acides aminés supplémentaire.

3. Protéine de fusion telle que revendiquée selon la revendication 2, dans laquelle ladite séquence antigénique est jointe directement ou indirectement au niveau de la terminaison N à une séquence GST ou séquence étiquette His.

4. Protéine de fusion telle que revendiquée selon la revendication 2 ou la revendication 3, dans laquelle ladite séquence antigénique est jointe à une ou plusieurs séquence(s) supplémentaire(s) contenant un épitope capables de développer une réponse d'anticorps ou une réponse cellulaire immune chez des êtres humains contre un antigène de *P. falciparum.*

5. Protéine de fusion telle que revendiquée selon la revendication 4, dans laquelle lesdites séquences supplémentaires contenant un épitope sont des séquences dérivées de MSP1 de *P. falciparum* choisies parmi (i) des séquences de cellule T humaine contenant un épitope capables d'induire une réponse cellulaire immune à *P. falciparum*; (ii) une séquence fournissant une région de répétition MSP1 bloc 2 du type MAD 20 ; et (iii) une séquence fournissant une région de répétition MSP1 bloc 2 du type R033.

6. Protéine de fusion telle que revendiquée selon la revendication 5, comprenant une construction hybride, dans laquelle une séquence antigénique telle que définie selon la revendication 1 est jointe au niveau de la terminaison N à une séquence fournissant un ou plusieurs épitope(s) de cellule T humaine dérivé(s) d'un allèle MSP1.

7. Protéine de fusion telle que revendiquée selon la revendication 6, comprenant une construction hybride constituée de (i) un allèle bloc 1-bloc 2 du type MSP1 K1 et (ii) une séquence antigénique telle que définie selon la revendication 1.

8. Protéine de fusion telle que revendiquée selon la revendication 6 ou la revendication 7, dans laquelle ladite construction hybride est en outre étendue par l'addition d'une séquence fournissant une région MSP1 bloc 2 du type RO33 et une région MSP1 bloc 2 du type MAD 20.

9. Protéine de fusion telle que revendiquée selon la revendication 8, dans laquelle ladite construction hybride est constituée des composants suivants dans le sens N- à C-terminal: (i) un allèle bloc 1-bloc 2 du type MSP1 K1; (ii) une séquence antigénique telle que définie selon la revendication 1; et (iii) une séquence fournissant une région MSP1 bloc 2 du type RO33 et une région MSP1 bloc 2 du type MAD 20.

10. Protéine de fusion telle que revendiquée selon la revendication 8, dans laquelle ladite construction hybride est constituée des composants suivants dans le sens N- à C-terminal: (i) une séquence fournissant un ou plusieurs épitope(s) de cellule T humaine dérivé(s) d'un allèle du type MSP1 K1; (ii) une séquence antigénique telle que définie selon la revendication 1 ; et (iii) une séquence fournissant une région MSP1 bloc 2 du type RO33 et une région MSP1 bloc 2 du type MAD 20.

11. Protéine de fusion telle que revendiquée selon l'une quelconque des revendications 6 à 10, dans laquelle ladite construction hybride est jointe en outre au niveau de la terminaison N à une séquence GST N-terminale ou une séquence étiquette His.

12. Polynucléotide qui code un polypeptide ou une protéine selon l'une quelconque des revendications précédentes.

13. Polynucléotide tel que revendiqué selon la revendication 12, qui est un ADN.

14. Vecteur comprenant un polynucléotide tel que revendiqué selon la revendication 13.

15. Polynucléotide tel que revendiqué selon la revendication 13, dans lequel la séquence codant pour ledit polypeptide ou ladite protéine est fonctionnellement liée à une séquence promotrice.

16. Vecteur d'expression comprenant un polynucléotide tel que revendiqué selon la revendication 15.

17. Procédé de production d'un polypeptide ou d'une protéine tel(le) que revendiqué(e) selon l'une quelconque des revendications 1 à 11, qui comprend la culture de cellules hôtes contenant un vecteur d'expression selon la revendication 16 sous des conditions par lesquelles ledit polypeptide ou ladite protéine est exprimé(e) et l'isolement dudit polypeptide ou de ladite protéine.

18. Cellule hôte *in vitro* contenant un vecteur d'expression selon la revendication 16.

19. Composition pour induire une réponse immune à *P. falciparum,* qui comprend un polypeptide ou une protéine selon l'une quelconque des revendications 1 à 11, conjointement à un diluant ou un adjuvant pharmaceutiquement acceptable.

20. Composition pour induire une réponse immune à *P. falciparum,* qui comprend un polynucléotide selon la revendication 15 ou un vecteur d'expression selon la revendication 16 conjointement à un support.

21. Utilisation d'un polypeptide ou d'une protéine tel(le) que revendiqué(e) selon l'une quelconque des revendications 1 à 11, un polynucléotide tel que revendiqué selon la revendication 15, ou un vecteur d'expression tel que revendiqué selon la revendication 16, dans la fabrication d'une composition pour l'utilisation dans l'induction d'une réponse immune à *P. falciparum.*

22. Procédé de typage de la région MSP1 bloc 2 dans un sérum provenant d'un individu infecté par *P. falciparum* qui comprend la mise en contact dudit sérum avec un polypeptide comprenant une séquence antigénique telle que définie selon la revendication 1 et la détermination si les anticorps dans ledit sérum se lient à ladite séquence.

23. Procédé de typage de la région MSP1 bloc 2 dans des schizontes cultivés de *P. falciparum* qui comprend la mise en contact des schizontes avec un sérum contenant des anticorps développés contre une séquence antigénique telle que définie selon la revendication 1 et la détermination de la liaison desdits anticorps auxdits schizontes.
